# EUROPEAN PATENT APPLICATION

(11) **EP 3 945 527 A1**
(43) Date of publication of application: **02.02.2022**
(21) Application number: 21187926.7
(22) Date of filing: 27.07.2021
(51) Int. Cl.: G16H 10/40, G16H 40/67

(54) **TEST RESULT AUTO VERIFICATION**

(30) Priority: 31.07.2020 US 202016944999
(71) Applicant: SYSMEX CORPORATION, Kobe-shi Hyogo 651-0073 (JP)
(72) Inventor: Jimenez, Ariel, Hyogo, 651-0073 (JP); Rodgers, Amanda, Hyogo, 651-0073 (JP); Diemeke, Robert, Hyogo, 651-0073 (JP); Dahlberg, Mark, Hyogo, 651-0073 (JP)
(74) Representative: Hoffmann Eitle

(57) **Abstract**

An analyzer analyzes patient samples and the test results are then validated. The validation of test results may be performed either remotely over a network or at a local computer. For example, while network communication errors may prevent remote validation, the local validation could still be performed, which allows auto validation to be continuously performed. The validation of the test results of the patient sample obtained by the analyzer may further be performed by: 1) the first rule included in the first system of the laboratory; and 2) the second rule included in the second system accessible remotely via the network. The present disclosure further relates to an inspection management method based on at least one of the two rules.

## Description

### BACKGROUND

Diagnostic analyzers, such as hematology analyzers, are utilized to perform various measurements of the constituents of a blood sample. Once a measurement is performed, a test result is generated from an analyzer. A test result generated by analyzers are needed to be verified before the test result is released. However, it would be quite burdensome if all those test result must be manually verified one by one. In order to assist the validation procedure in a laboratory, a middleware for performing auto-verification has been commercially available to date. Typical middleware is provided in a laboratory and stores multiple auto verification rules to run on a test result. If laboratory-defined acceptance criteria established by the rules is satisfied by the test result, the test result is automatically validated and released to a lab network, such as a laboratory information system (LIS), for reporting. If the test result fails to meet the criteria, the test result is held for manual review. If any action-triggering rule is met, a predetermined action such as Rerun test or Reflex test is automatically initiated. To ensure verification is possible, any error in communication must be avoided. Errors in communication can result in the loss of data transmission including analyzer test results and may prevent proper verification procedure of those results. Other problems for laboratory verification will become apparent upon reading the descriptions of the various embodiments described below.

### SUMMARY

This disclosure relates generally to auto verification of test results obtained with an analyzer device for analyzing patient samples. The verification of test results may be performed either remotely over a network or at a local computer. For example, while network communication errors may prevent remote verification, the local verification could still be performed, which enables auto verification to be continuously performed.

The verification of the test results of the patient sample obtained by the analyzer may be performed by: 1) the first rule included in the first system accessible remotely via the network; and 2) the second rule included in the second system of the laboratory. The present disclosure further relates to a verification method based on at least one of the two rules. According to the present embodiments, even when it is not possible to connect to the cloud in which the latest verification rule is held, by applying the verification rule held in the laboratory, it is possible to continuously perform auto verification.

In one embodiment, a method implemented by a computing system including first and second systems for managing a test by a diagnostic analyzer, comprises: obtaining a test result of a sample from the diagnostic analyzer in a laboratory; and verifying the test result according to at least one of: 1) a first rule executable by the first system which is accessible from the laboratory via a network; or 2) a second rule executable by the second system deployed in the laboratory.

In another embodiment, a system for managing a test of a patient sample, comprises: an analyzer for generating the test result from the patient sample; a cloud system configured to verify the test result remotely upon receiving the test result and to communicate the a verification result; and a local system configured to verify the test result locally when the cloud system cannot receive the test result, cannot verify the test result, or cannot communicate the verification.

In another embodiment, a system for managing a test of a patient sample includes an analyzer for generating the test result from the patient sample, a local system configured to communicate with the analyzer and receive the test result, and a cloud system configured to verify the test result, wherein the verification of the test result is not performed when a predetermined condition is identified, further wherein the local system is configured to perform the verification when the predetermined condition is identified..

### BRIEF DESCRIPTION OF THE DRAWINGS

The system and method may be better understood with reference to the following drawings and description. Non-limiting and non-exhaustive embodiments are described with reference to the following drawings. The components in the drawings are not necessarily to scale, emphasis instead being placed upon illustrating the principles of the invention. In the drawings, like referenced numerals designate corresponding parts throughout the different views.
Figure 1a is a block diagram illustrating an example laboratory communication system.
Figure 1b is a block diagram illustrating example communication with a local/cloud system.
Figure 2 is a block diagram illustrating another example laboratory communication system.
Figure 3 is a block diagram illustrating a local manager in an example laboratory communication system.
Figure 4 is a block diagram illustrating an example cloud system.
Figure 5 illustrates an example user interface for rule definition.
Figure 6 illustrates another example user interface for rule definition.
Figure 7 illustrates another example user interface for rule definition.
Figures 8A is a flow chart illustrating a dual-sites verification workflow example.
Figures 8B is a flow chart illustrating a second dual-sites verification workflow example.
Figure 8C is a flow chart illustrating a backup verification workflow example.
Figure 8D is a flow chart illustrating another backup verification workflow example.
Figure 8E is a flow chart illustrating another backup verification workflow example.
Figure 8F is a flow chart illustrating another backup verification workflow example.
Figure 8G is a flow chart illustrating another backup verification workflow example.
Figure 8H is a flow chart illustrating another backup verification workflow example.
Figure 9 is a flow chart illustrating another backup verification workflow example.
Figure 10 is a flow chart illustrating a collaborative verification workflow example.
Figure 11 is a flow chart illustrating another collaborative verification workflow example.
Figure 12 illustrates examples of an IP message.
Figure 13 illustrates example auto verification rules.

### DETAILED DESCRIPTION

By way of introduction, the disclosed embodiments relate to systems and methods for verifying test results from tests with a diagnostic analyzer in a laboratory setting. An analyzer analyzes patient samples to generate test results and the test results are then verified. In the present disclosure, the verification of test results may be performed either remotely over a network or at a local computer or both. In one example, while network communication errors may prevent remote verification, the local verification could still be performed, which allows auto verification to be continuously performed. The verification of the test results of the patient sample obtained by the analyzer may further be performed by: 1) the first rule included in the first system of the laboratory; and 2) the second rule included in the second system accessible remotely via the network. The present disclosure further relates to a method for verifying test result based on at least one of the two rules.

Reference will now be made in detail to exemplary embodiments of the invention, examples of which are illustrated in the accompanying drawings. When appropriate, the same reference numbers are used throughout the drawings to refer to the same or like parts. The numerous innovative teachings of the present application will be described with particular reference to presently preferred embodiments (by way of example, and not of limitation). The present application describes several inventions, and none of the statements below should be taken as limiting the claims generally.

For simplicity and clarity of illustration, the drawing figures illustrate the general manner of construction, and description and details of well-known features and techniques may be omitted to avoid unnecessarily obscuring the invention. Additionally, elements in the drawing figures are not necessarily drawn to scale, some areas or elements may be expanded to help improve understanding of embodiments of the invention.

The word 'couple' and similar terms do not necessarily denote direct and immediate connections, but also include connections through intermediate elements or devices. For purposes of convenience and clarity only, directional (up/down, etc.) or motional (forward/back, etc.) terms may be used with respect to the drawings. These and similar directional terms should not be construed to limit the scope in any manner. It will also be understood that other embodiments may be utilized without departing from the scope of the present disclosure, and that the detailed description is not to be taken in a limiting sense, and that elements may be differently positioned, or otherwise noted as in the appended claims without requirements of the written description being required thereto.

The terms "first," "second," "third," "fourth," and the like in the description and the claims, if any, may be used for distinguishing between similar elements and not necessarily for describing a particular sequential or chronological order. It is to be understood that the terms so used are interchangeable. Furthermore, the terms "comprise," "include," "have," and any variations thereof, are intended to cover non-exclusive inclusions, such that a process, method, article, apparatus, or composition that comprises a list of elements is not necessarily limited to those elements, but may include other elements not expressly listed or inherent to such process, method, article, apparatus, or composition.

The aspects of the present disclosure may be described herein in terms of functional block components and various processing steps. It should be appreciated that such functional blocks may be realized by any number of hardware and/or software components configured to perform the specified functions. For example, these aspects may employ various integrated circuit components, e.g., memory elements, processing elements, logic elements, look-up tables, and the like, which may carry out a variety of functions under the control of one or more microprocessors or other control devices.

Similarly, the software elements of the present disclosure may be implemented with any programming or scripting languages with the various algorithms being implemented with any combination of data structures, objects, processes, routines, or other programming elements. Further, it should be noted that the present disclosure may employ any number of conventional techniques for data transmission, signaling, data processing, network control, and the like.

The particular implementations shown and described herein are for explanatory purposes and are not intended to otherwise be limiting in any way. Furthermore, the connecting lines shown in the various figures contained herein are intended to represent exemplary functional relationships and/or physical couplings between the various elements. It should be noted that many alternative or additional functional relationships or physical connections may be present in a practical incentive system implemented in accordance with the disclosure.

As will be appreciated by one of ordinary skill in the art, aspects of the present disclosure may be embodied as a method or a system. Furthermore, these aspects of the present disclosure may take the form of a computer program product on a tangible computer-readable storage medium having computer-readable program-code embodied in the storage medium. Any suitable computer-readable storage medium may be utilized, including hard disks, CD-ROM, optical storage devices, magnetic storage devices, and/or the like. These computer program instructions may be loaded onto a general purpose computer, special purpose computer, or other programmable data processing apparatus to produce a machine, such that the instructions which execute on the computer or other programmable data processing apparatus create means for implementing the functions specified in the flowchart block or blocks. These computer program instructions may also be stored in a computer-readable memory that can direct a computer or other programmable data processing apparatus to function in a particular manner, such that the instructions stored in the computer-readable memory produce an article of manufacture including instruction means which implement the function specified in the flowchart block or blocks. The computer program instructions may also be loaded onto a computer or other programmable data processing apparatus to cause a series of operational steps to be performed on the computer or other programmable apparatus to produce a computer-implemented process such that the instructions which execute on the computer or other programmable apparatus provide steps for implementing the functions specified in the flowchart block or blocks.

The terms "verify" or "verification" include a process of executing predetermined set of rules, which is established by a laboratory, against a test result to determine if the test result satisfies laboratory-defined acceptance criteria. "Autoverification" or "auto verification" is a process for automatically verifying test results based on the predetermined set of rules. In autoverification, test results generated by an analyzer interfaced to a laboratory network, such as a laboratory information system (LIS), are compared by computer software against laboratory-defined acceptance parameters or action triggering parameters. If results fall within the acceptance parameters, they are automatically validated and released for reporting with no additional human intervention.

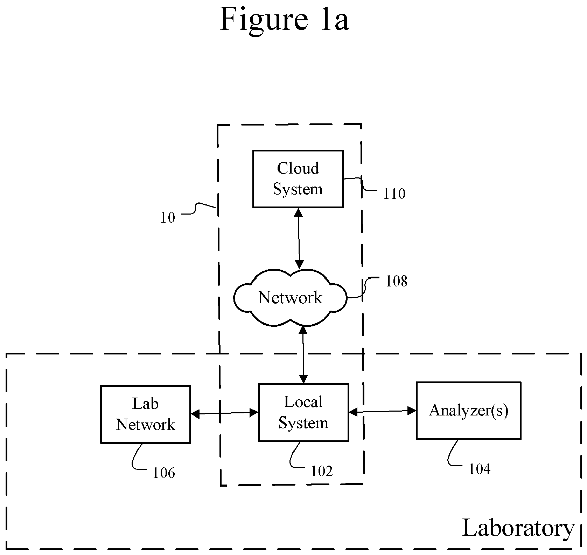

The terms "validate" or "validation" include a process for identifying a test result as being ready for reporting. Results that fall outside of these defined acceptance parameters may be reviewed by a medical laboratory scientist prior to reporting. When a test result falls within the action triggering parameters, a predefined action such as an additional testing order is created. Figure 1a is a block diagram illustrating an example laboratory communication system. In one aspect of the present disclosure relates to a workflow management system (WMS) 10 as illustrated in Figure 1a. In particular, the laboratory system may include diagnostic analyzer(s) 104 ("analyzer") that perform a measurement on a clinical sample collected from a patient. In one embodiment, the analyzer(s) 104 output test results of samples (e.g., blood) that include several parameters (e.g., numbers of red blood cells, white blood cells, platelets, etc.). The analyzer(s) 104 may count a number of blood cells in tests but the accuracy of the test results should be verified or validated to determine if the test was sufficient or if the test should be rerun. This may include determining whether abnormal test results came from the sample or was caused by the analyzer(s) 104. The verification may be through auto verification using one or more rules, which are a set of criteria for the verification/validation.

The analyzers 104 may be physically located in the laboratory. In the laboratory, there may be one or more analyzers 104 for performing sample measurements and obtaining test results. The test results from the analyzer(s) 104 may be verified by either the local system 102 or the cloud system 110. The verification process may include executing rules on the test results. If the verification fails according to the rules established, the test performed by the analyzer(s) 104 may need to be manually reviewed by a technician for manual validation or there may be a need of running additional test such as Rerun test or Reflex test as further described below.

The analyzer(s) 104 may be any type of diagnostic analyzer. The type of analyzer is not limited but may be in vitro diagnostic analyzer which is configured to perform tests on in vitro samples collected from a patient. In another example, the analyzer is a blood cell counter configured to count blood cells. In another example, the analyzers may include a smear preparing apparatus which is configured to smear a blood on a microscopic slide for visual examination. In one embodiment in which the analyzers include a smear preparing apparatus, the analyzers may further include a slide imaging analyzer which is configured to image a smeared sample on the microscopic slide and analyze cells by digitally analyzing images of cells. The sample is, in one embodiment, a blood sample. The blood sample may be whole blood, blood serum, or blood plasma. In other embodiments, the sample may be other types, which may be collected from a patient, such as body fluid or urine.

The workflow management system 10 may include a local system 102 which is deployed in a laboratory. The workflow management system 10 may include a cloud system 110 which is remotely located from the laboratory and is communicably coupled with the local system 102 over a network 108. The local system 102 may be a computing device that performs a verification of the test results locally. Conversely, the cloud system 110 may perform a verification of test results remotely. The cloud system 110 may be accessible from a web browser deployed in a user terminal such as a computer, PC, tablet, smartphone, etc. The cloud system 110 may be accessible by multiple laboratories (users can check test results with a browser) and accessible from anywhere. Further, the cloud system 110 can consolidate multiple test results from multiple laboratories. However, if the could system 110 is unavailable, rather than stopping verification, that process may be performed locally. A user can manage a rule for verifying a test result. The user also can manually validate the test result by accessing the cloud system 110. The cloud system 110 may be deployed in a data center.

The lab network 106 may also be referred to as the Laboratory Information System or LIS. The lab network 106 is a computer system that helps to manage many aspects of a medical laboratory and integrates data from throughout a laboratory. In one embodiment, the validated measurements or data from the analyzers is sent to the lab network 106 for future access. In other embodiments, the lab network 106 assists with managing the laboratory, including inputting, processing, and storing the information and data of the laboratory. For example, when patient goes to the laboratory to get his/her blood drawn and tested, the lab network 106 helps to test orders. If the network of the laboratory happens to be down, the lab network 106 cannot receive verification results from the cloud system 110. As described, the local system 102 can also perform the verification so the lab network 106 can still receive verification results.

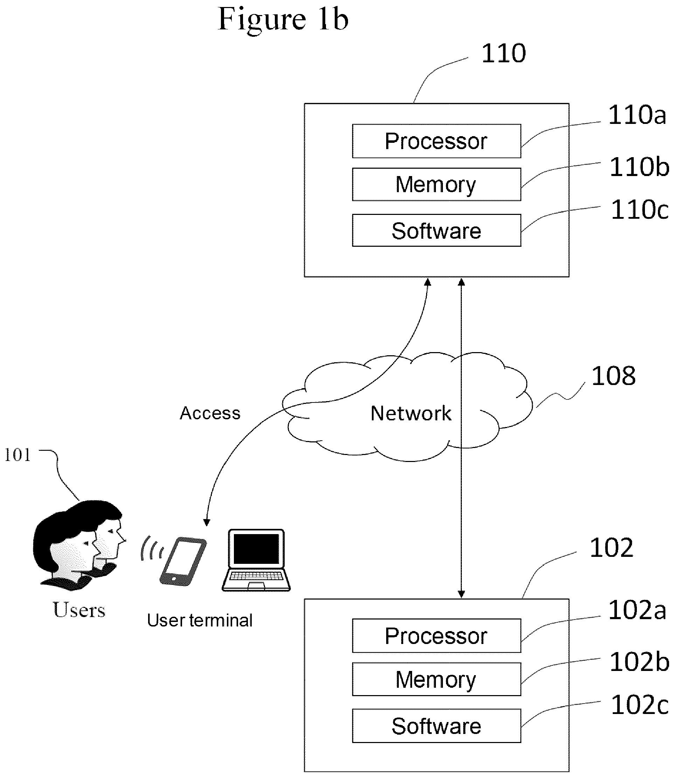

Figure 1b is a block diagram illustrating example communication with a local/cloud system. The system in Figure 1b illustrates one embodiment for communication of a user 101 with the cloud system 110. In particular, Figure 1b illustrates example components of the local system 102 and the cloud system 110. As shown in Figure 1a and other figures, the local system 102 may be local to the laboratory, while the cloud system 110 may be remote and accessed via a network, such as the network 108.

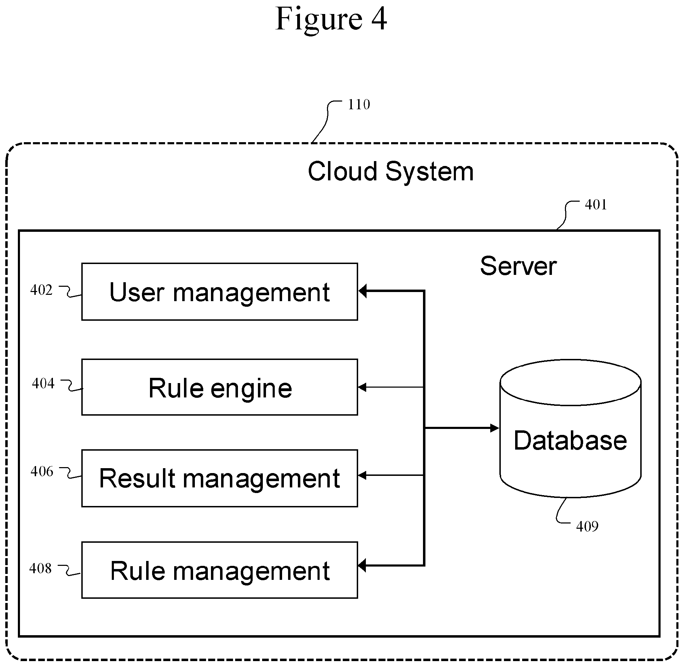

The users may be laboratory workers accessing the validated test results. There may be different users that access the lab network 106 and the analyzer(s) 104 or it may be the same users 101 for each component. The communication by the user 101 with the cloud system 110 may be through the network 108. User can access the cloud system 110 by using a web browser or other software of a user terminal. The cloud system 110 is further described below with respect to Figure 4.

The local/cloud system 102/110 may include or be part of a computing device. As described, the local/cloud system 102/110 may provide verification of test results from the analyzer(s) 104. The processing for the verification may be performed using the components illustrated in Figure 1b. In these embodiments, the local/cloud system 102/110 may be implemented in software that is run by a computing device. The local/cloud system 102/110 may be any hardware or software used for performing the functions described herein. The cloud system 110 may include a processor 110a, a memory 110b, and software 110c. The local system 102 may include a processor 102a, a memory 102b, and software 102c.

The user terminal may be a computer, PC, tablet or smartphone and may include a user input device, a display, or a camera which constitutes a user interface. The user input device may include a keyboard, keypad or a cursor control device, such as a mouse, or a joystick, touch screen display, remote control or any other device operative to allow a user or administrator to interact with the cloud system 110. The display of the user terminal may act as an interface for the user to see the functioning of the processor 110a, or as an interface with the software 110c/102c for validating test data.

The processors 110a/102a in the local/cloud system 102/110 may include a central processing unit (CPU), a graphics processing unit (GPU), a digital signal processor (DSP) or other type of processing device. The processors 110a/102a may be a component in any one of a variety of systems. For example, the processors 110a/102a may be part of a standard personal computer or a workstation. The processors 110a/102a may be one or more general processors, digital signal processors, application specific integrated circuits, field programmable gate arrays, servers, networks, digital circuits, analog circuits, combinations thereof, or other now known or later developed devices for analyzing and processing data. The processors 110a/102a may operate in conjunction with a software program (i.e. software 110c/102c), such as code generated manually (i.e., programmed). The software 110c/102c may include the functions described below for verifying test results from analyzers. The functions described below for the local/cloud system 102/110 may be implemented at least partially in software (e.g. software 110c/102c) in some embodiments.

The processors 110a/102a may be coupled with the memory 110b/102b, or the memory 110b/102b may be a separate component. The software 110c/102c may be stored in the memory 110b/102b. The memory 110b/102b may include, but is not limited to, computer readable storage media such as various types of volatile and non-volatile storage media, including random access memory, read-only memory, programmable read-only memory, electrically programmable read-only memory, electrically erasable read-only memory, flash memory, magnetic tape or disk, optical media and the like. The memory 110b/102b may include a random access memory for the processor 110a/102a. Alternatively, the memory 110b/102b may be separate from the processor 120, such as a cache memory of a processor, the system memory, or other memory. The memory 110b/102bb may be an external storage device or database for storing recorded tracking data, or an analysis of the data. Examples include a hard drive, compact disc ("CD"), digital video disc ("DVD"), memory card, memory stick, floppy disc, universal serial bus ("USB") memory device, or any other device operative to store data. The memory 110b/102b is operable to store instructions executable by the processor 120.

The functions, acts or tasks illustrated in the figures or described herein may be performed by the programmed processor executing the instructions stored in the software 110c/102c or the memory 110b/102b. The functions, acts or tasks are independent of the particular type of instruction set, storage media, processor or processing strategy and may be performed by software, hardware, integrated circuits, firm-ware, micro-code and the like, operating alone or in combination. Likewise, processing strategies may include multiprocessing, multitasking, parallel processing and the like. The processors 110a/102a are configured to execute the software 110c/102c.

The present disclosure contemplates a computer-readable medium that includes instructions or receives and executes instructions responsive to a propagated signal, so that a device connected to a network can communicate voice, video, audio, images or any other data over a network. Instructions from user terminal are sent over the network and inputted to the cloud system 110 via a communication port. The communication port may be created in software or may be a physical connection in hardware. The communication port may be configured to connect with a network, external media, display, or any other components in the system of Figures 1a/1b, or combinations thereof. The connection with the network may be a physical connection, such as a wired Ethernet connection or may be established wirelessly as discussed below. Likewise, the connections with other components of the systems of Figures 1a/1b may be physical connections or may be established wirelessly.

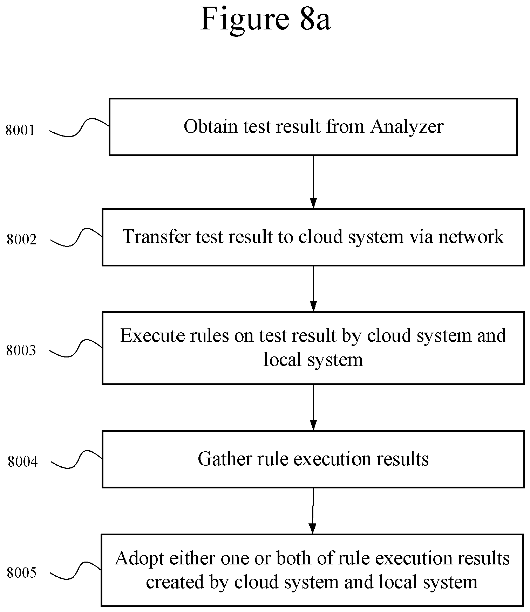

Although not shown, data used by the local/cloud system 102/110 may be stored in locations other than the memory 110b/102b, such as a database connected through the network 108. For example, the images that are acquired may be stored in the memory 110b/102b and/or stored in a database accessible via the network 108. Likewise, the machine learning model may be operated by the local/cloud system 102/110 but may include functionality stored in the memory 110b/102b and/or stored in a database accessible via the network 108. The local/cloud system 102/110 may include communication ports configured to connect with a network. The network or networks that may connect any of the components in the systems of Figures 1a/1b to enable communication of data between the devices may include wired networks, wireless networks, or combinations thereof. The wireless network may be a cellular telephone network, a network operating according to a standardized protocol such as IEEE 802.11, 802.16, 802.20, published by the Institute of Electrical and Electronics Engineers, Inc., or WiMax network. Further, the network(s) may be a public network, such as the Internet, a private network, such as an intranet, or combinations thereof, and may utilize a variety of networking protocols now available or later developed including, but not limited to TCP/IP based networking protocols. The local/cloud system 102/110 performs the operations described in the embodiments herein. For example, Figures 8a-9 illustrate example functions performed by the local/cloud system 102/110.

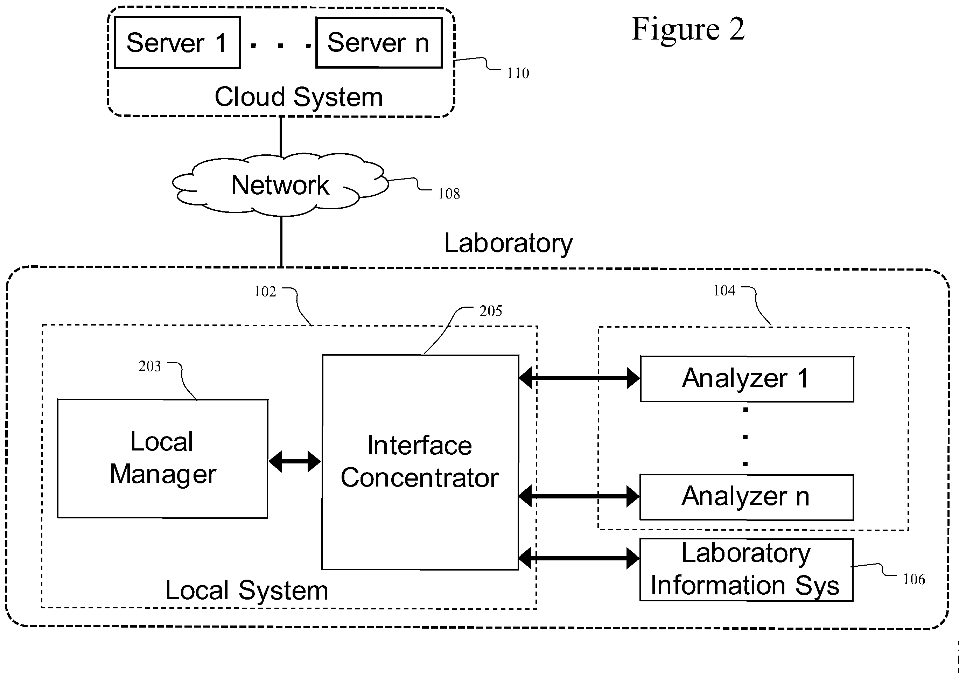

Figure 2 is a block diagram illustrating another example laboratory communication system. Figure 2 illustrates a similar embodiment to the laboratory system shown in Figure 1a. Figure 2 illustrates that the cloud system 110 connected over the network 108 to the laboratory. The laboratory includes the local system 102, the analyzer(s) 104, and the laboratory network 106, also referred to as the laboratory information system (LIS). Figure 2 further illustrates that the local system 102 may include a local manager 203 that acts as an agent for transferring data back and forth between an interface concentrator 205 and a server from the cloud system 110. The function of the local manager 203 may be realized by software as further discussed with respect to Figure 1b. The local manager 203 may be the same device having the function of the interface concentrator 205, or may be a separate device. The interface concentrator 205 may simplify interfaces between analyzer(s) 104 and the lab network 106 by managing communications with the analyzer(s) 104 and by minimizing the lab network 106 connectivity to a single interface in one embodiment. The single interface may use ASTM or HL7 protocol. The function of the interface concentrator 205 may be realized by a software in a device as discussed with respect to Figure 1b.

The cloud system 110 in Figure 2 is illustrated as having one or more servers. In particular, Figure 2 illustrates n servers in the cloud system 110. The server may be a main component of the cloud system 110. The server may be located in the data center. The function of the server is further described below with respect to Figure 4. For example, the server may have functions that allows users to manage the test results effectively, such as defining rules to mark test results that need attention, validating the test results.

Figure 2 also illustrates that the analyzer(s) 104 can be one or more analyzers and shows n analyzers. Each of the n analyzers communicates with the local system 202 through the interface concentrator 205. The analyzer(s) 104 include detection methods/channels for analyzing a sample and performing tests on the sample.

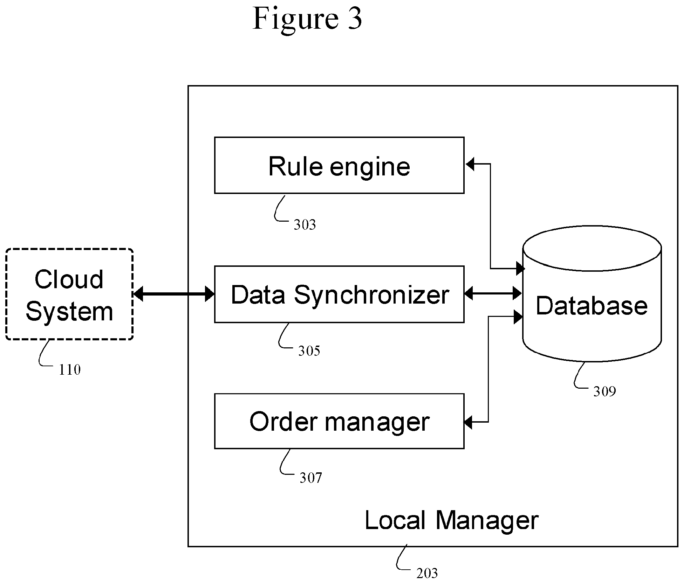

Figure 3 is a block diagram illustrating a local manager 203 in an example laboratory communication system. Figure 3 illustrates an example communication between the cloud system 110 and the local manager 203. The local manager 203 may include a rule engine 303 that runs auto verification rules to validate the test result from the analyzers 104. The rule engine 303 retrieves the rule(s) from the data base 309. The rule engine 303 may issue, as a result of rule execution, a Rerun test order or Reflex test order according to one of the rules. The local manager 203 may include a data synchronizer 305 that transfers data to the server(s) of the cloud system 110. The data synchronizer 305 also receives data from the cloud system 110. The data synchronizer 305 may also: 1) transfer the test result from the analyzer(s) 104 to the cloud system 110; 2) receive a verification result including validated test result from the cloud system 110; 3) receive a Rerun/Reflex test order from the cloud system 110; and/or 4) synchronize the verification rules defined at the cloud system 110. In one embodiment, the data synchronizer 305 is programmed to copy all data of the test results that are received from the analyzer(s) 104 and stored in the database 309 to the database 409 of the cloud system 110 (discussed with respect to Figure 4). In one embodiment, the data synchronizer 305 is programmed to replicate all of the verification rules defined at the cloud system 110 and stores the replicated rules in the database 309.

The data synchronizer 305 may also transfer/receive the data to/from the cloud system 110 automatically and without user intervention. An IP address may be assigned to the local manager 203. The cloud system 110 recognizes the IP address assigned to the local manager 203. The cloud system 110 then allows access via the IP address. The data synchronizer 305 uses the IP address to communicate with the cloud system 110. The data synchronizer 305 may operate in a background to be automated and without user intervention, or manipulation of the analyzer(s) 104 and/or the lab network 106. The data synchronizer 305 may be able to transfer/receive the data without user manipulation.

The local manager 203 may also include an order manager 307. The order manager 307 manages the test order, which may be issued by the lab network 106 and the cloud system 110, which may issue the Rerun/Reflex order according to the rule. The order manager 307 sends, to the analyzer(s) 104, the test order received from lab network 106. The order manager 307 sends, to the analyzer(s) 104, the Rerun/Reflex test order. The Rerun/Reflex test order may be issued by the cloud system 110, or may be issued by the rule engine 303.

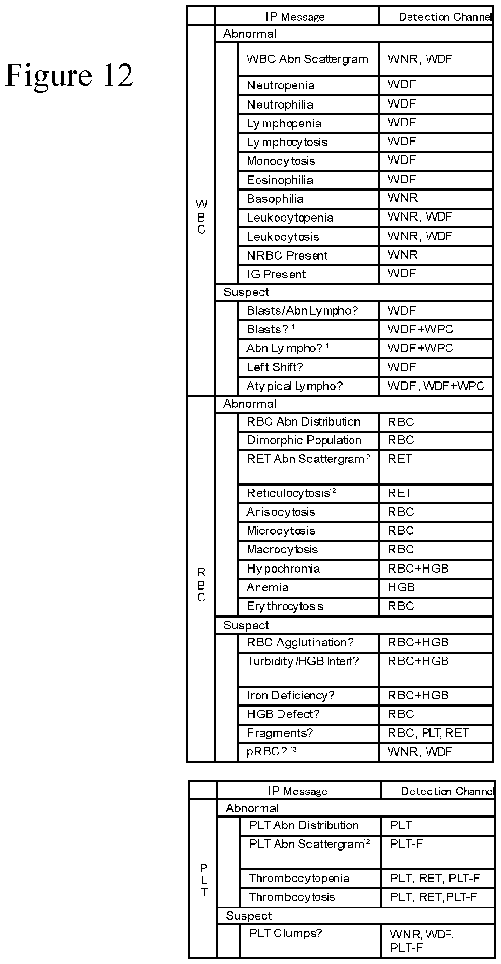

The database 309 may store information or data relevant for the tests from the analyzer(s) 104 and/or for the verification of those test results. In particular, the database 309 may store test result data which may include:
- Sample ID: The identifier of the patient sample.
- Patient ID: The identifier of the patient associated with the patient sample identified by the sample ID.
- Name: The name of the patient associated with the patient identified by the patient ID.
- Gender: Gender of the patient associated with the patient identified by the patient ID.
- Date of Birth (DOB): Date of birth of the patient associated with the patient identified by the patient ID.
- Attending doctor: Name or identifier of the attending doctor of the patient identified by the patient ID.
- Collection date/time: The date/time that patient provide the sample.
- Receipt date/time: The date/time that the sample is delivered to the laboratory.
- Test parameters: This information may be a list of test parameters (e.g., "WBC", "RBC"...). This information may be a text data which has a code of the test parameters each divided by a delimiter (e.g., "|", tab, ",").
- Result: This information may be a list of results for each of the test parameters (e.g., "WBC", "RBC"...). This information may be a text data which has a the code of the test parameters and a numerical value corresponding to the code. Each of the combination of the code and value may be divided by the delimiter (e.g., "|", tab, ","). • IP message: This information may be a text, code or the like for identifying some messages/alert issued by the analyzer(s) 104. Figure 12 illustrates further examples of an IP message.
- Reflex request: This information identifies whether the reflex test is requested or not. This information is added by completing auto verification.
- Reflex test code : This information may be a list of reflex test parameters (e.g., "WBC", "RBC"...). This information may be a text data which has a code of the test parameters each divided by a delimiter (e.g., "|", tab, ",").
- Rerun request: This information identifies whether the rerun test is requested or not. This information is added by completing auto verification.
- Rerun test code: This information may be identical to the "test parameters" of original test. The user may select other test parameters.
- Graphic: Graphic data of Histogram\Scattergram of the test result.

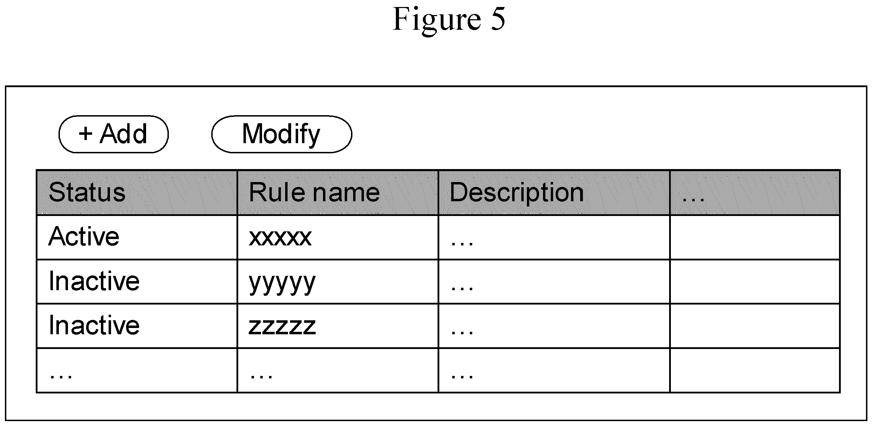

In addition, the database 309 may store rule information. Figures 5-7 illustrate graphical user interfaces for rule entry/modification. The database 309 may include the following information for each rule:
- Rule name: A text to identify a rule.
- Inactive/active flag: A flag-type information to indicate whether a corresponding rule is active or inactive.
- Description: A text of a detailed explanation of a corresponding rule.
- Patient age: A patient demographic info to be used for triggering the rule. This information relates to an age of the patient. This info may be a range of patient age (e.g., from 20 to 30 years old).
- Sample age: A patient demographic info to be used for triggering the rule. This info indicates a duration which has progressed from a patient sample is taken. This info may be a range of the sample age.
- Patient Location: A patient demographic info to be used for triggering the rule. This info may identify a location of the patient. The location may be a ward, care unit etc.
- Gender: A patient demographic to be used for triggering the rule. This info identifies a gender of the patient.
- Test: Information to identify a test parameter relating to a test-related condition for triggering the rule.
- Low condition: This is a lower limit condition of the test parameter identified by the "Test". This may be a numerical value with a character such as "<", ">", "=<", "=>".
- High Condition: This is an upper limit condition of the test parameter identified by the "Test". This may be a numerical value with a character such as "<", ">", "=<", "=>".
- Test of previous result: A previous test result may be used as a trigger of the rule. The "Test of previous result" identifies a test parameter relating to a condition of the previous test.
- Rerun order: This info is used to identify an action triggered by the rule. This info may be a flag-type info, or used to identify whether a Rerun test is ordered according to a rule-triggering condition.
- Test parameters: This info identifies test parameters of the Rerun test. The parameters may be identical to that of the test order, which causes the Rerun order. A user may be able to identify test parameters, which differ from that of the test order which causes the Rerun order.
- Priority: This info is used to identify a priority of the Rerun order.
- Reflex order: This info to be used to identify an action triggered by the rule. This info may be a flag-type info. This info is used to identify whether a Reflex test is ordered according to a rule-triggering condition.
- Delta Check Alert: This info to be used to identify an action triggered by the rule. This info may be a flag-type info. This info may be used to identify whether a delta check alert to be popped-up as a warning message.

Figure 4 is a block diagram illustrating an example cloud system. The cloud system 110 may be the same as the cloud system illustrated in Figures 1a-3 or may be a different cloud system. As discussed with respect to Figure 2, the cloud system 110 may include one or more servers. Figure 4 illustrates an embodiment with a server 401. The server includes a database 409. In some embodiments, the database 409 stores different data from the database 309 from the local manager 203. In other embodiments, there may be synchronization of at least some data and rules.

The server 401 includes a rule engine 404 that runs auto verification rules on test results and validates the test result according to rule(s). The rule engine 404 retrieves the rule from the database 409. The rule engine 404 may issue the Rerun/Reflex test order according to the rule. The server 401 may also include user management module 402 that manages user accounts which are allowed to access to the cloud system 110. The user management module 402 manages a user's login according to account information (e.g. user name, password, etc.) registered in the database 409. When a user accesses to the cloud system 110 for logging in his/her account through a web browser of a user terminal, the user management module 402 requires a user ID and a password to allow the user to log in. In response to an input of the user ID and the password, the user management module 402 verifies a user based on the user ID and the password. The rule management module 408 manages rules to be executed by the rule engine 404. The rule management module 408 registers the defined rules into the database 409. The rule management module 408 provides a Graphical User Interface (GUI) to user who logging in the cloud system 110, such as the interfaces shown in Figures 5-7. Users are allowed to define the rules through the example GUIs shown in Figures 5-7. The server 401 includes result management module 406 which may provide another GUI which allows a user to manage the test results (including the validated test results). Users may also be able to manually validate the test results via the GUI.

The database 409 may store the same or similar test result data as with the database 309 discussed above since the data synchronizer 305 is programmed to copy all data stored in the database 309 to the database 409. In addition, the database 409 may store the same verification rules data as in database 309 since the data synchronizer 305 is programmed to replicate all verification rules stored in the databased 409 as discussed above. Remote access to the database 409 may require additional security measures. The information may be specific to a corresponding user account (e.g., "Account ID") so that the information is only accessible by a user having the corresponding account. Any attempted access from other users is prohibited. The user management 402 may control this accessibility and security of the user accounts.

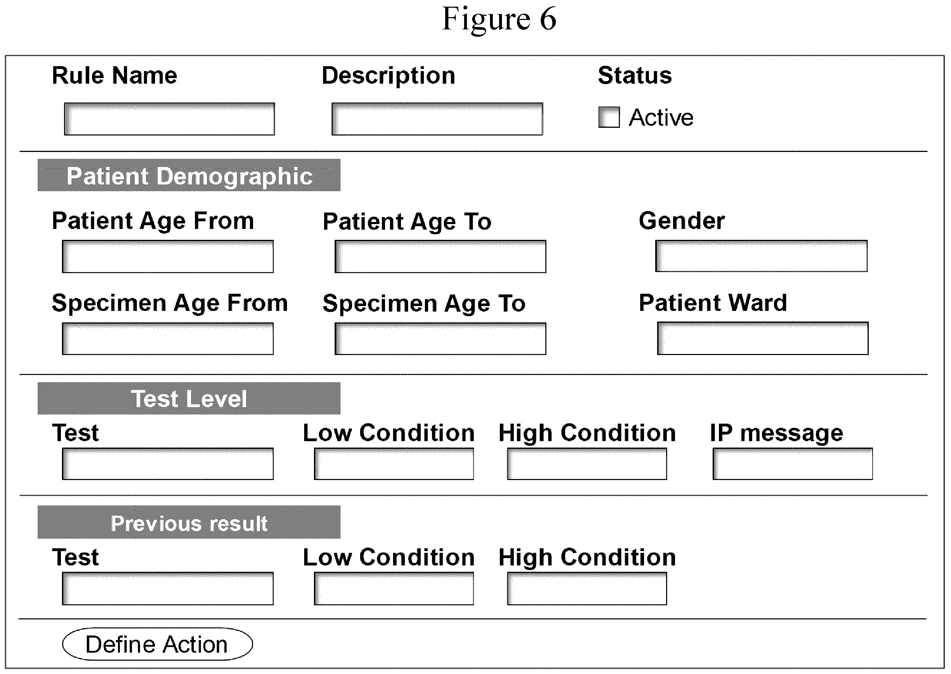

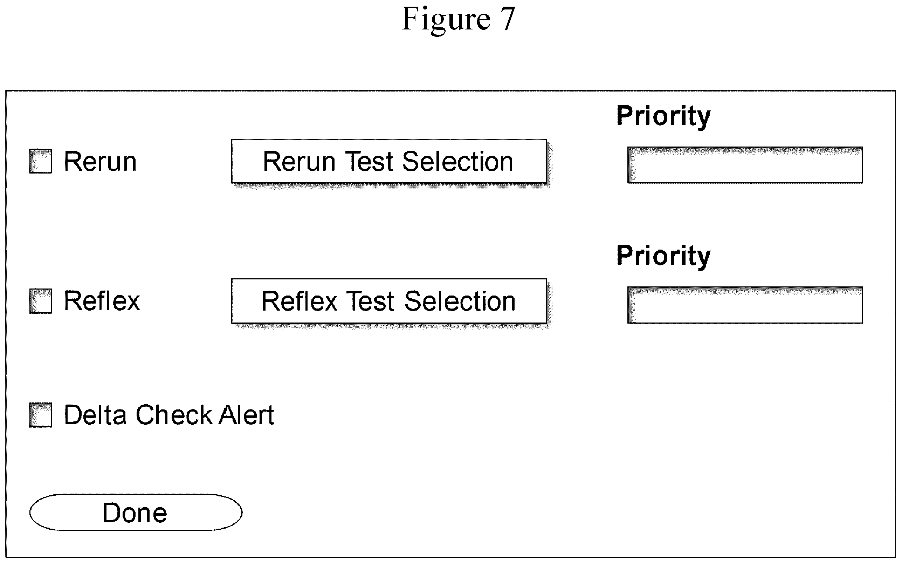

Figures 5-7 illustrates example graphical user interfaces (GUI) for rule definition that may be accessible over the cloud. Figure 5 illustrates an example user interface for rule definition. A user can add or modify a particular rule. The rule may be identified by a rule name and include a status and description. Figure 6 illustrates another example user interface for rule definition. Figure 6 illustrates defining a patient demographic, a test level, and/or a previous result as part of the rule definition. Figure 7 illustrates another example user interface for rule definition that illustrates the rerun and reflex selection.

Hereinafter, several examples of workflow realized by the present embodiments will be described.

### Dual-Sites Verification Workflow

Figure 8A illustrates a dual-sites verification workflow. In this example, the workflow management system 10 performs rule execution both at the cloud system 110 and the local system 102 (i.e. mirroring). In particular, in block 8001, the local system 102 obtains the test result from the analyzer(s) 104. The local system 102 stores the test result in the database 309. In block 8002, the data synchronizer 305 of the local system 102 retrieves the test result from the database 309 and transfers the test result to the cloud system 110 over the network 108. Once the cloud system 110 receives the test result from the local system 102, the cloud system 110 stores the test result in the database 409. In block 8003, the rule engine 404 of the cloud system 110 performs rule execution on the test result to verify the test result. In block 8003, the rule engine 303 of the local system 102 performs rule execution on the same test result to verify. This execution may be in parallel between the two systems.

The auto verification rules stored in the local system 102 and the cloud system 110 are synchronized periodically or every time a new rule is created or any rule is modified by the user. Thus, the auto verification rules in the local system 102 and the cloud system 110 are similar or the same as one other. Therefore, the outcomes of the rule execution at the local system 102 and the cloud system 110 should match one another in one embodiment. However, in an example situation in which one of the auto verification rules refers to a site-specific information, which is not shared between the local system 102 and the cloud system 110, the outcome may be different. For example, as one of the site-dependent information, the cloud system 110 stores multiple test results which may be obtained through previous tests for a same patient. If one of the rules is defined in relation to the previous test result, the local system 102 may not be able to refer to the previous test result and thus cannot complete the rule execution or the verification result may be insufficient. In another example, as one of the site-specific information, the local system 102 stores analyzer information such as quality control data or reagent expiration data. If one of the rules is defined in relation to the analyzer information to evaluate the reliability of the test result by referring to the analyzer information, the cloud system 110 may not be able to complete the rule execution. According to this dual-sites verification workflow, the local system 102 and the cloud system 110 can mutually supplement the verification procedure to complete the rule execution.

In block 8004, the data synchronizer 305 of the local system 102 transfers the rule execution result to the cloud system 110. The rule execution results coming from the local system 102 includes, but is not limited to, the information of: 1) whether the rule execution was completed successfully; 2) whether the test result is validated or not; and 3) triggered rule; 4) actions to be taken (e.g., additional tests). The cloud system 110 compares the rule execution results received from the local system 102 with the rule execution result created by the rule engine 409 to determine which of the results should be adopted. If both results are identical, it means that the site-specific information made no difference on the rule execution result, and therefore the cloud system 110 can adopt either one. On the contrary, if the rule execution results are different, the cloud system 110 must select one of the results according to any rule. For example, if one of the verification result is successful while the other verification result is not successful, the cloud system 110 may adopt the successful one. In another example, if one of the rule execution result shows that the test result should be validated while the other result shows that one of the rules is triggered and defined action should be taken, the cloud system 110 may adopt the latter for safety. In another example, if one of the verification result made by cloud system 110 shows that Rule A is triggered while the other verification result made by the local system 102 shows that Rule B is triggered, the cloud system 110 may adopt the verification result showing a rule with higher priority is triggered. In one embodiment, if Rule A is prioritized over Rule B, the verification result of Rule A may be adopted. In another example, the cloud system 110 may adopt both of the verification results, which means that the cloud system performs both actions triggered by Rule A and Rule B.

In the aforementioned example, the blocks 8004 and 8005 are carried out at the cloud system 110, however the disclosure is not limited to such configuration. The local system 102 may carry out the operations instead of the cloud system 110.

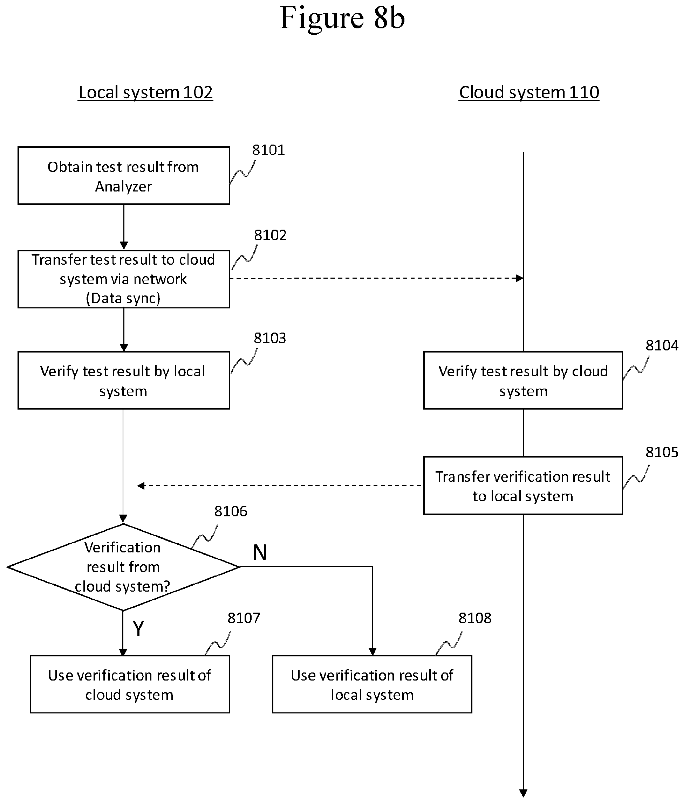

Figure 8B illustrates another example of a dual-sites verification workflow. In block 8101, the local system 102 obtains the test result from the analyzer(s) 104. The local system 102 stores the test result in the database 309. In block 8102, the data synchronizer 305 of the local system 102 performs data synchronization with the cloud system 110. Although Figure 8B illustrates that the block 8102 is performed only after the block 8101, this is only for the purpose of simplifying the illustration. In some embodiments, the data synchronization happens periodically or every time a data update in the database 309 or database 409 occurs.
Through the data synchronization at block 8102, the test result stored in the database 309 is transferred or at least attempted to be transferred to the cloud system 110 over the network 108. When the network if functioning properly and the communication between the local system 102 and the cloud system 110 is working, the data sync should be carried out and the test results arrive at the cloud system 110. Once the cloud system 110 receives the test result from the local system 102, the cloud system 110 stores the test result in the database 409 and the rule engine 404 of the cloud system 110 performs rule execution on the test result to verify the test result in block 8104. In block 8105, the cloud system 110 transfers or at least attempts to transfer the verification result to the local system 102. In block 8103, the rule engine 303 of the local system 102 performs rule execution on the same test result to verify. This verification may be in parallel.

In block 8106, the local system 102 determines if the verification result from the cloud system 110 is returned. In particular, the local system 102 determines whether the verification result made by the cloud system 110 is returned in a predetermined time period from the transfer of the test result to the cloud system 110 in block 8102. When a predetermined time passes without receiving the verification result from the cloud system 110, the local system proceeds to block 8108 and uses the verification result made by the local system 102, namely, the verification result created at the block 8103. If the verification result from the cloud system 110 arrives within the predetermined time period, the local system 102 proceeds to block 8107 and uses the verification result from the cloud system 110, namely, the verification result created at block 8104. In this instance, the verification result created by the local system 102 at the block 8103 is discarded or replaced.

In this example workflow, the rule engine 404 of the cloud system 110 works as a primary rule engine while the rule engine 303 of the local system 102 keeps working in background, regardless of the network connection status between the cloud system 110 and the local system 102. According to the configuration, even if the communication between the cloud system 110 and the local system 102 becomes disabled or unstable, auto verification can still be able to continue by using the rule engine 303.

### Backup Verification Workflow

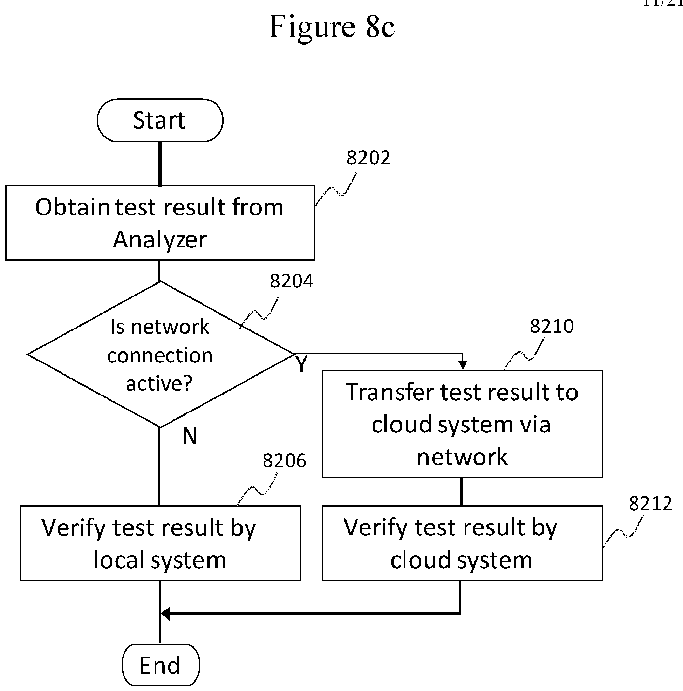

Figure 8C is a flow chart illustrating an example of a backup verification workflow. Figure 8C is an illustrative overview of the workflow performed by the workflow management system 10. The operations conducted by the local system 102 and the cloud system 110 will be described later in greater detail. In one embodiment, the local system 102 obtains test results from the analyzer(s) 104 in block 8202. A determination is then made, by the local system 102, as to whether the network connection is active in block 8204. If the network connection is active, the verification can be performed by the cloud system 110 in blocks 8210, 8212. Otherwise, the test result must be verified locally in block 8206. Assuming the connection is active and stable in block 804, the test result is transferred from the local system 102 to the cloud system 110 via the network 108 in block 810. The test result can then be verified by the cloud system 110 in block 812. If the network connection is not active or stable in block 8204, the verification is performed locally by the local system 102 in block 8206.

Figure 8C illustrates that the verification by the local system 102 is a backup verification process. In an instance where the cloud system 110 is unavailable, the local system 102 functions as a backup verification mechanism.

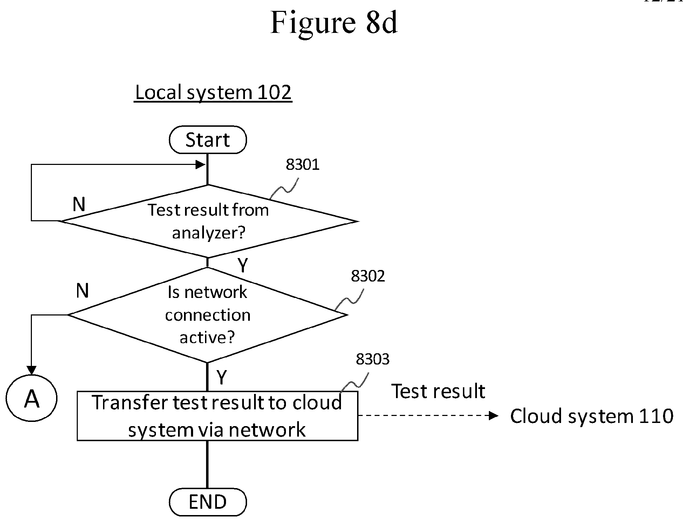

Figure 8D through 8H illustrates detailed operations carried out by the local system 102 and the cloud system 110 to realize the backup verification workflow illustrated in Figure 8C. In Figure 8D, the local system 102 determines whether the local system 102 receives a test result from analyzer(s) 104 in block 8301. If a test result from analyzer(s) 104 is received at the local system 102, the processor 120 of the local system 102 advances the process to block 8302. If test result is not received at the local system 102 in block 8301, the process is repeated until the determination in block 8301 turns to be positive (YES). In block 8302, the local system 102 determines whether the network connection between the cloud system 110 is active. The local system 102 is able to monitor the connection status by sending out data toward the cloud system 110 and watching if a response returns in a predetermined time period. If no response comes back from the cloud system 110, the local system determines that the network connection is not active. In a typical situation, when the Internet network outage occurs outside a laboratory, the determination in block 8302 turns to be negative (No). If the network connection is active, the processor 102a of the local system 102 transfers the test result received from the analyzer(s) 104 to the cloud system 110 over the network in block 8303. If the network is not active, the processor 102a of the local system 102 proceeds to the process illustrated in Figure 8D, which is described below.

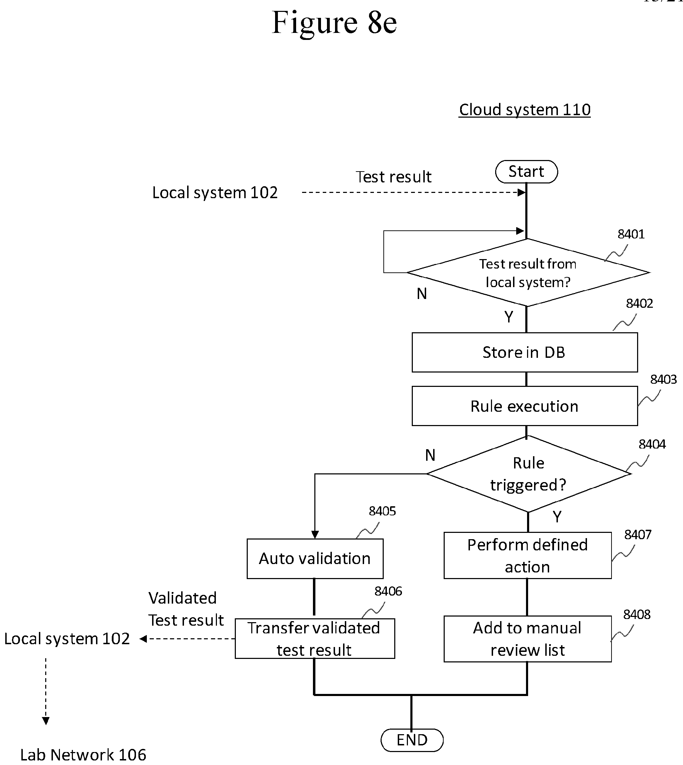

In Figure 8E, the server 401 of the cloud system 110 determines whether the server 401 has received the test result from the local system 102 in block 8401. If the cloud system 110 has received the test result from the local system 102, the result management module 406 of the cloud system 110 stores the test result received in the database 409. If the test result is not received at the cloud system 110, the cloud system 110 repeats the determination in block 8401. In block 8403, the rule engine module 404 of the server 401 performs rule execution on the test result. In the rule execution step, the rule engine module 404 compares the test result with active rules stored in the database 409. Active rule may refer to a rule whose status is set "active" like the one illustrated in the top row of Figure 5. Upon comparing the target test result with the active rules individually in prioritized order, the rule engine module 404 goes through all of rules to determine if the test result satisfies any condition of any one of the rules. In block 8404, the rule engine module 404 determines whether any verification rule was triggered in the rule execution process of block 8404. If no rules were triggered, the cloud system 110 validates the test result in block 8405. In particular, the result management module 406 of the server 401 sets a flag of "validated" to the data of the test result stored in the database 409. The flag signifies that the test result has been confirmed to be reliable and it is ready to be released to the lab network 106. As illustrated in dotted lines in Figure 8E, the validated test result is first transferred to the local system 102 and then re-transferred to the lab network 106 by the local system 102.

In this example, a test result is validated when no rule is triggered through the verification process (rule execution). In other words, the verification rule executed at the block 8403 is constructed to rule out a test result which needs a manual review or an additional action. A test result is validated if it is not ruled out by any of the rules. This is only one example. Criteria for validating a test result may be another form. For example, the rule engine 404 may use an alternative rule to positively identify a test result to be validated.

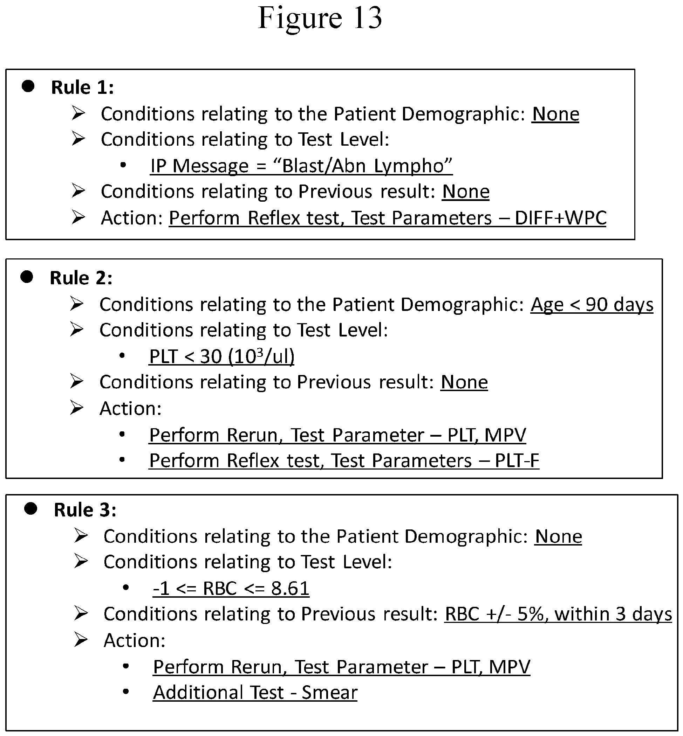

Referring back to block 8404, if any one of the rules has been triggered, the result management module 406 carries out predefined action according to the triggered rule. Figure 13 illustrates exemplary rules. In the Rule 1, the action to be taken is defined as "Perform Reflex test, Test Parameters - DIFF+WPC" which means a Reflex order should be created for the test parameters of DIFF + WPC if the conditions are met. Assuming that the Rule 1 was triggered in block 8404, the result management module 406 of the cloud system 110 creates Reflex test order to run additional test of DIFF and WPC for the same sample in block 8207. Similarly, if the Rule 2 was triggered in block 8404, the result management module 406 creates Rerun test order for parameters of PLT and MPV and Reflex test order to run additional test of PLT-F. If the Rule 3 was triggered in block 8404, the result management module 406 creates Rerun test order for parameters of PLT and MPV and smear preparing order to create a blood smear for the sample. Once a new order is created by the result management module 406, the order is transferred to the local system 102 over the network and the local system forward the order to the analyzer(s) 104. The analyzer(s) 104 are programmed to automatically run the ordered test on the designated sample as instructed by the order. In block 8408, the result management module 406 adds the sample ID of the test result in a manual review list stored in the database 409. The manual review list is a list of sample IDs in which sample IDs of test results triggering any rules are listed. The manual review list is accessible for user by accessing the server through a web browser. The user can track test results through the sample ID and see the test result to see if the test result can be validated.

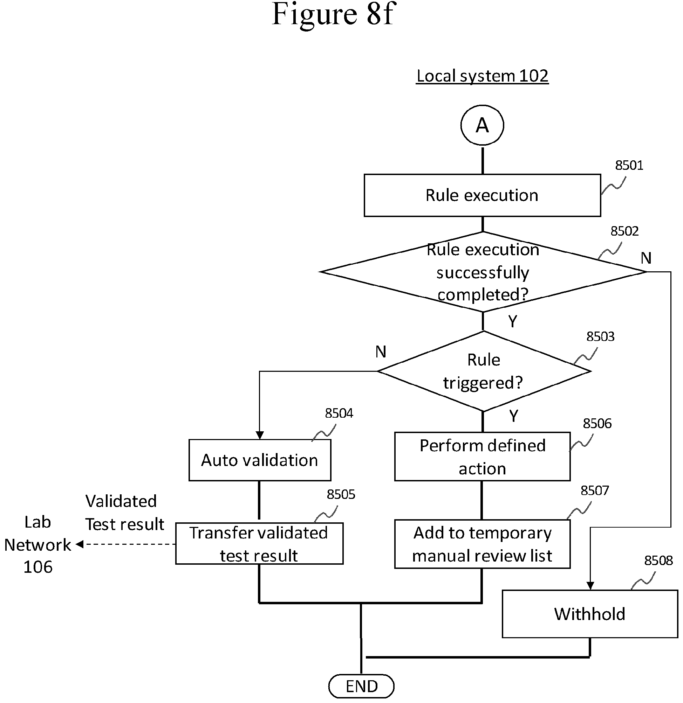

Figure 8F illustrates the steps to be taken by the local system 102 with a network disruption. In block 8501, the local system 102 performs rule execution locally. As mentioned above, the local system 102 includes a data synchronizer 305 which synchronizes the auto verification rules defined at the cloud system 110. Every time a new rule is defined or any existing rule is modified on the cloud system 110, they are replicated to the local system 102 and stored in the database 309. As such, the rules defined on the cloud system 110 and the rules stored in the local system 102 are synchronized. Therefore, even if any network disruption occurs, the local system 102 can still continue autoverification with the latest set of rules in block 8501. The rule execution in block 8501 is performed in the same way as block 8403 in Figure 8E.

Although all the rules defined on the cloud system 110 are copied to the local system 102, depending on the sample or on the test result, there may be instances where rule execution cannot be successfully completed. For example, with reference to Rule 3 of Figure 13, Rule 3 includes a condition relating to previous result. In this example, if the parameter of RBC in the test result changes over ±5% compared to the previous test result, the rule is triggered. However, when the cloud system 110 retains previous test results, the local system 102 may not retain the data of previous test result. In that example, the local system 102 alone cannot compare the test result with the previous test result, and therefore, the Rule 3 may not be run by the rule engine 303 of the local system 102.

The local system 102 determines whether the rule execution by the rule engine 303 completed successfully in block 8502. If the determination result was negative (No) at block 8502, in other words, if there was at least one rule which was unable to execute at the local system 102 like in the situation mentioned immediately above, the local system 102 proceeds to block 8508 to withhold the test result from further action. Withholding herein means that the processor 120 sets aside the test result until the network connection is restored. Once the network connection is restored, the withheld test results are back into a process, as described below.

If the determination was positive (Yes) at block 8502, the local system 102 further determines whether any rule was triggered in block 8503. If no rules were triggered, the local system 102 validates the test result in block 8504 and transfers the validated test result to LIS 106. The steps of block 8504 and 8505 are substantially same with the steps performed in blocks 8405 and 8406.

Referring back to block 8503, if any one of the rules has been triggered, the local system 102 carries out predefined action according to the triggered rule. The detailed steps performed in block 8506 is substantially same with block 8407. In block 8507, the local system 102 adds the sample ID of the test result in a temporary manual review list which is temporarily created in the database 309. This temporary manual review list is, similarly to the manual review list already explained with reference to block 8408, a list of sample IDs in which sample IDs of test results triggering any rules are listed. This temporary manual review list is integrated to the manual review list of the cloud system 110 after the network connection is restored.

As long as the network disruption continues, the operations of Figure 8F are continuously carried out. As such, auto verification by the local system 102 can be performed locally even during the network disruption, and therefore the laboratory operation is not needed to be stopped.

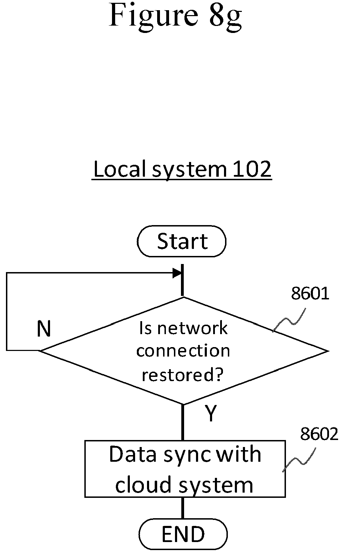

Figure 8G illustrates the operations carried out by the local system 102 after the network disruption occurs. The local system 102 determines whether the network connection is restored in block 8601. The local system 102 is programmed to attempt to establish a network connection with the cloud system 110 when the network connection between the local system 102 and the cloud system 110 is disputed. The local system 102 continues the attempt until it succeeds. If the determination is positive (Yes) in block 8601, in other words, if the local system 102 confirmed that the network connection is restored, the data synchronizer 305 of the local system 102 starts data synchronization with the cloud system 110 in block 8602. In the data sync process, data processed or created by the local system 102 during the network disruption is transferred to the cloud system 110. The data may include: 1) the test results received from the analyzer(s) 104 during the network disruption; 2) auto verification results; 3) information of rules executed; 4) orders created by the rule engine 303; and 5) temporary manual review list. The data may also include the test results withheld by the local system 102 at the block 8508.

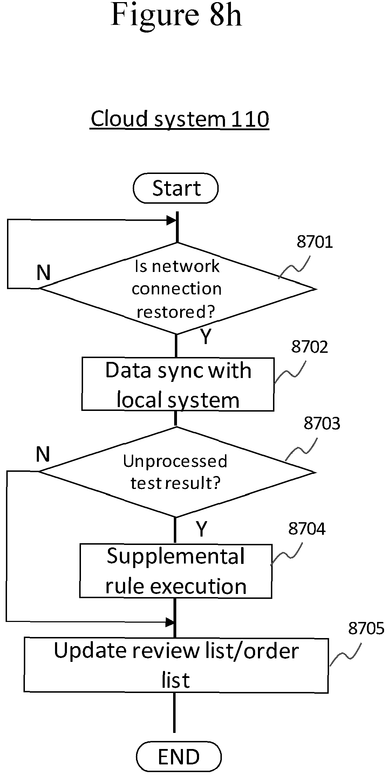

Figure 8H illustrates the operations carried out by the cloud system 110 after the network disruption occurs. As mentioned above, the local system 102 is programmed to continue attempting to establish a network connection with the cloud system 110. In block 8701, the cloud system 110 determines whether the network connection is restored. If the network connection is restored, the cloud system 110 performs data synchronization with the local system 102. In particular, the cloud system 110 receives the data transferred from the local system 102 and store the data in the database 409. In block 8702, the cloud system 110 determines if there is unprocessed test result among the data transferred from the local system 102. In particular, the cloud system 110 checks if any test result was withheld by the local system 102. As mentioned, the local system 102 may not be able to complete rule execution on some test results because of lacking of data (e.g., previous test result). In such a case, the determination in block 8703 turns to be positive (Yes) and the cloud system 110 proceeds to block 8704. If the cloud system 110 determines that all the test results received from the local system at block 8702 are successfully completed with the rule execution, the determination in block 8703 turns to be negative (No) and the cloud system 110 proceeds to block 8705, skipping block 8704. In block 8704, the cloud system 110 performs supplemental rule execution. Supplemental rule execution is to run rules by the rule engine 404 on the test results to which the local rule execution by the rule engine 303 failed. For example, if the cloud system 110 receives, in block 8702, a test result which the rule engine 303 withheld due to incapability of performing auto verification for the reason of lack of previous test result, the cloud system 110 performs complete rule execution on the test result by referring to the previous test result. In block 8705, the cloud system 110 updates the manual review list and order list by incorporating the test results and the auto verification results. Users are allowed to access to the test results in the manual review list through a web browser and manually validate them as described herein.

### Collaborative Verification Workflow

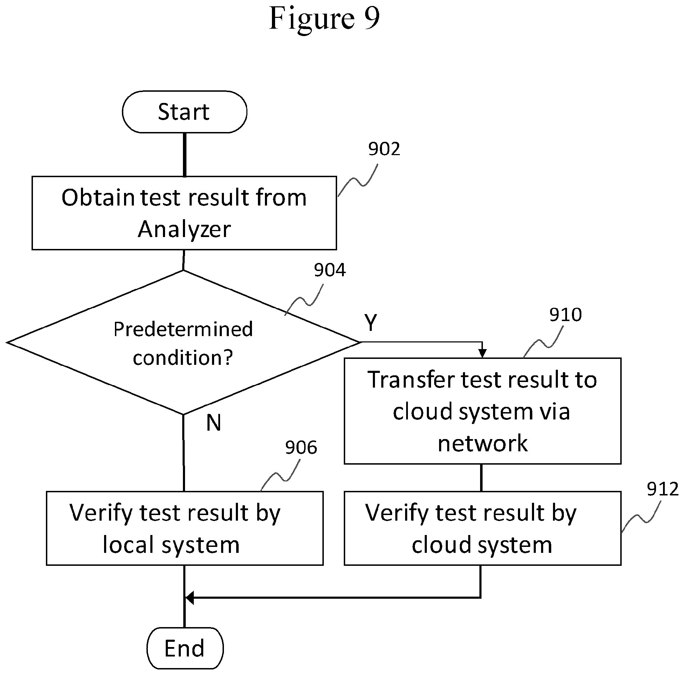

Figure 9 illustrates an alternative embodiment in which the verification procedure is collaboratively performed by the local system 102 and the cloud system 110. In this embodiment, a predetermined condition in which the local system 102 is used for verification rather than verifying with the cloud system 110 is set. In this embodiment, a first set of auto verification rules ("first set or rules") executable by the rule engine 404 of the cloud system 110 and a second set of auto verification rules ("second set or rules") executable by the rule engine 303 of the local system 102 may be different. The first set of rules and the second set of rules may be entirely different or at least partially different. In other words, one or more rules included in the first set of rules may be same or equivalent with one or more of the rules included in the second set of rules.

In one embodiment, the predetermined condition is satisfied if the test result obtained from the analyzer(s) 104 includes an analyzer flag. In this embodiment, the first set of rules may be for verifying test results with no flags. The second set of rules may be for verifying test results with flags. One example of the flag may be IP message as illustrated in Figure 12. Figure 12 illustrates examples of an IP message. The IP messages may be messages/alert issued by the analyzer(s) 104 when the analyzer(s) 104 detects any abnormality or suspected finding of a sample as a result of running a preprogrammed analysis algorithm embedded in the analyzer(s) 104. The IP message may be included in test result data or may be attached to the test result as metadata. IP message indicates that there may be something wrong with the test result, in turn, it suggests that the test result with IP message is likely to trigger additional testing (e.g., Rerun or Reflex) or manual review. Conversely, it also suggests that the test result without IP message is less likely to trigger additional test or manual review. Therefore, by determining whether the test result has flags or not, the test results can be sorted out according to likelihood of triggering additional testing and/or manual review.

In block 904, the test result with flag is transferred to the cloud system 110 and auto verification is performed by the rule engine 404 of the cloud system 110. According to this configuration, auto verification rule specifically designed to process the test result having flags can be constructed on the cloud system 110 and, if needed, a complex rule set suitably designed to process the suspect sample may be established. In return, the rule set on the local system 102 may be simplified.

In another embodiment, the first set of rules is defined to verify test results of regular samples while the second set of rules is defined to verify test results of irregular samples. Regular sample herein refers to a sample which is collected from a patient for routine health checkup or a routine testing. Typical regular sample is stored in a blood collection tube attached with sample ID. Irregular sample refers to a sample which is collected in irregular basis or a sample collected in irregular way. Examples of the irregular samples may include a manually-handled sample, pediatric blood sample, blood sample with low volume, and/or a sample to be tested urgently.

Figure 9 is a flow chart illustrating one example of the collaborative verification workflow. The local system 102 obtains the test results from the analyzer(s) 104 in block 902. A determination is then made, by the local system 102, as to whether the predetermined condition is satisfied in block 904. In one embodiment, the determination in block 904 determines whether the test result is of a regular sample. If the determination is positive (Yes), the verification can be performed by the cloud system 110 in blocks 910, 912. If the determination is negative (No) which means the test result is of irregular sample, or must be verified locally in block 906. If the predetermined condition is satisfied in block 904, the local system 102 transfers the test result to the cloud system 110 via the network 108 in block 910. The test result can then be verified by the cloud system 110 in block 912. If the predetermined condition is not satisfied in block 904, the verification is performed locally by the local system 102 in block 906.

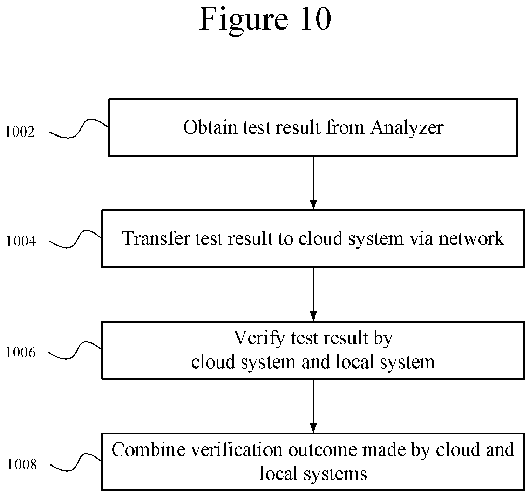

Figure 10 is a flow chart illustrating another embodiment of collaborative verification workflow. In this example, auto verification is performed at both the cloud system 110 and the local system 102 and the verification results are combined to derive a single outcome.

The test result is obtained from the analyzer(s) 104 by the local system 102 in block 1002. The test result is then transferred to the cloud system 110 via the network 108 in block 1004. The test result may be verified by both the cloud system 110 and the local system 102 in block 1006. The verification results from both the cloud system 110 and the local system 102 can be combined in block 1008. The combination of the verification results may be carried out by the local system 102 or the cloud system 110. In an example where the local system 102 combines outcomes, the cloud system 110 transfers the verification result to the local system 102 upon completion of the auto verification at block 1006. In the example where the cloud system 110 combines outcomes, the local system 102 transfers the verification result to the cloud system 110 upon completion of the auto verification at block 1006.

Examples of combination of verification results are further described below. In one example of combining the verification from block 1008, the rule engine 303 of local manager 203 determines no action is to be taken, but the rule engine 404 of the cloud system 110 determines at least one action (e.g., perform a Reflex test or a Rerun) should be taken. In this example, the combined verification would be to proceed with the Reflex test.

In another example of combining the verification from block 1008, the rule engine 303 of local manager 203 determines Reflex test (DIFF) as the action, but the rule engine 404 of the cloud system 110 determines Reflex test (DIFF+WPC). In this example, the combined verification result would be to proceed with the Perform Reflex test (DIFF+WPC).

In another example of combining the verification result from block 1008, the rule engine 303 of local manager 203 determines Rerun test (e.g. Rerun all test parameters tested in an original test) as the action, but the rule engine 404 of the cloud system 110 determines a Reflex test (PLT-F). In this example, the combined verification would be to proceed with the Rerun test and Reflex test (PLT-F).

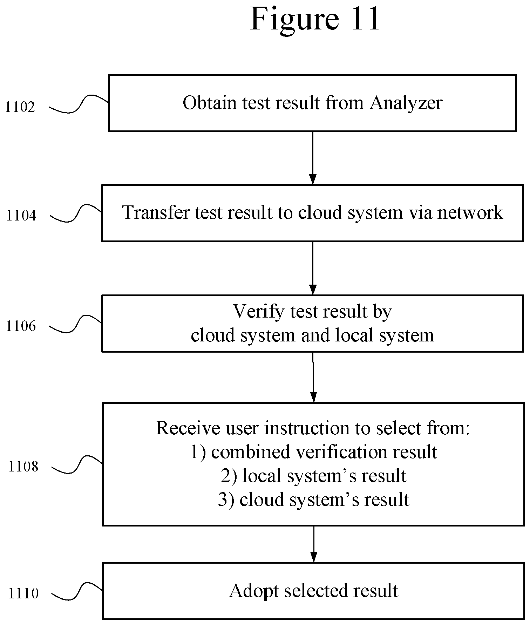

In the examples above, the combined verification may error on the side of caution and following the recommendation that is the most conservative. If there is a change of any problem with the test result, it is safer and more accurate to proceed with correcting the test results. When the local system 102 and the cloud system 110 differently verifies the test result (as in the above examples), the result management 406 may provide a GUI which allows the user to select the outcome. In other words, the user selects the course of action when the local system 102 and the cloud system 110 provide different outcomes as shown in Figure 11.

Figure 11 is a flow chart illustrating another collaborative verification example. The test result is obtained from the analyzer(s) 104 by the local system 102 in block 1102. The test result is then transferred to the cloud system 110 via the network 108 in block 1104. The test result may be verified by both the cloud system 110 and the local system 102 in block 1106. The verification result made by the local system 102 is transferred to the cloud system 110. The user may be presented with the GUI on the user terminal to select verification results from: 1) combined verification result, 2) local system's verification result or 3) cloud system's verification result in block 1108. The cloud system 110 receives user selection in block 1108 and adopts the selected result in block 1110. In this embodiment, the local system 102 may be the main verification, while the cloud system 110 is the backup verification. The verification from both the cloud system 110 and the local system 102 can be either selected and/or combined in block 1110.

In some embodiments, the local system 102 may be configured to run different tests from the cloud system 110. Rather than functioning as a backup, the local system 102 may be a check on the cloud system 110 or may run entirely different and/or complementary tests/rules as the cloud system 110.

The system and process described above may be encoded in a signal bearing medium, a computer readable medium such as a memory, programmed within a device such as one or more integrated circuits, one or more processors or processed by a controller or a computer. That data may be analyzed in a computer system and used to generate a spectrum. If the methods are performed by software, the software may reside in a memory resident to or interfaced to a storage device, synchronizer, a communication interface, or non-volatile or volatile memory in communication with a transmitter. A circuit or electronic device designed to send data to another location. The memory may include an ordered listing of executable instructions for implementing logical functions. A logical function or any system element described may be implemented through optic circuitry, digital circuitry, through source code, through analog circuitry, through an analog source such as an analog electrical, audio, or video signal or a combination. The software may be embodied in any computer-readable or signal-bearing medium, for use by, or in connection with an instruction executable system, apparatus, or device. Such a system may include a computer-based system, a processor-containing system, or another system that may selectively fetch instructions from an instruction executable system, apparatus, or device that may also execute instructions.

A "computer-readable medium," "machine readable medium," "propagated-signal" medium, and/or "signal-bearing medium" may comprise any device that includes stores, communicates, propagates, or transports software for use by or in connection with an instruction executable system, apparatus, or device. The machine-readable medium may selectively be, but not limited to, an electronic, magnetic, optical, electromagnetic, infrared, or semiconductor system, apparatus, device, or propagation medium. A non-exhaustive list of examples of a machine-readable medium would include: an electrical connection "electronic" having one or more wires, a portable magnetic or optical disk, a volatile memory such as a Random Access Memory "RAM", a Read-Only Memory "ROM", an Erasable Programmable Read-Only Memory (EPROM or Flash memory), or an optical fiber. A machine-readable medium may also include a tangible medium upon which software is printed, as the software may be electronically stored as an image or in another format (e.g., through an optical scan), then compiled, and/or interpreted or otherwise processed. The processed medium may then be stored in a computer and/or machine memory.

The illustrations of the embodiments described herein are intended to provide a general understanding of the structure of the various embodiments. The illustrations are not intended to serve as a complete description of all of the elements and features of apparatus and systems that utilize the structures or methods described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The above disclosed subject matter is to be considered illustrative, and not restrictive, and the appended claims are intended to cover all such modifications, enhancements, and other embodiments, which fall within the true spirit and scope of the present invention. Thus, to the maximum extent allowed by law, the scope of the present invention is to be determined by the broadest permissible interpretation of the following claims and their equivalents, and shall not be restricted or limited by the foregoing detailed description. While various embodiments of the invention have been described, it will be apparent to those of ordinary skill in the art that many more embodiments and implementations are possible within the scope of the invention. Accordingly, the invention is not to be restricted except in light of the attached claims and their equivalents.

## Claims

1. A method implemented by a computing system including first and second systems for managing a test by a diagnostic analyzer, comprising:
obtaining a test result of a sample from the diagnostic analyzer in a laboratory; and
verifying the test result according to at least one of: 1) a first rule executable by the first system which is accessible from the laboratory via a network; or 2) a second rule executable by the second system deployed in the laboratory.

2. The method according to claim 1, further comprising:
sending, to the first system, the test result which is to be verified by the first rule.

3. The method according to claim 1, further comprising:
consolidating the verified test result on at least one of the first system or the second system.

4. The method according to claim 1, further comprising:
allowing a user to access the verified test result from a web browser.

5. The method according to claim 1, further comprising:
allowing a user to define the first rule via a web browser.

6. The method according to claim 1, further comprising:
handling the order and the test result via the first system which is an interface between the diagnostic analyzer and a laboratory network.

7. The method according to claim 1, further comprising:
handling the test result via the second system which establishes a connection to the first system.

8. The method according to claim 1, further comprising:
transferring, via the second system, the test result to the first system without user intervention.

9. The method according to claim 8, further comprising:
providing a Graphical User Interface (GUI) which allows a user to manage the test result transferred to the first system.

10. The method according to claim 1, further comprising:
synchronizing data relating to the test result between the first system and the second system.

11. The method according to claim 1, further comprising:
issuing an additional test order according to the verification of the test result.

12. The method according to claim 11, further comprising:
transferring, via the second system, the issued additional test order sent from the first system to the analyzer.

13. The method according to claim 1, further comprising:
synchronizing the first rule and the second rule between the first system and the second system.

14. The method according to claim 1, further comprising:
synchronizing the first rule and the second rule between the first and second systems; and
executing the second rule by the second system under a predetermined condition.

15. The method according to claim 1, further comprising:
synchronizing the first rule and the second rule between the first system and the second system; and
executing the second rule by the second system when a network connection between the laboratory and the first system is disabled.

16. The method according to claim 1, further comprising:
determining whether the verification with the second rule is successful; and
adopting the verification result by the first rule when the verification with the second rule is determined to be unsuccessful.

17. The method according to claim 16, wherein the verification with the second rule is determined to be unsuccessful when the second rule requires a certain data of the test result and the certain data is not stored in the second system.

18. The method according to claim 17, wherein the certain data is a previous test result originates from a same patient with the test result.

19. A system for managing a test by a diagnostic analyzer in a laboratory, the system comprising:
a cloud system, located remotely from the laboratory, configured to verify a test result remotely upon receiving the test result from the analyzer and to communicate a verification result; and
a local system configured to verify the test result locally.

20. A system for managing a test of a patient sample, the system comprising:
an analyzer for generating the test result from the patient sample;
a local system configured to communicate with the analyzer and to verify the test result with a first rule; and
a cloud system configured to receive the test result from the local system and to verify the test result with a second rule.
